# EUROPEAN PATENT APPLICATION

(11) **EP 3 248 587 A1**
(43) Date of publication of application: **29.11.2017**
(21) Application number: 17172850.4
(22) Date of filing: 24.05.2017
(51) Int. Cl.: A61K 8/25, A61K 8/26, A61Q 1/10, A61K 8/73, A61K 8/81, A61K 8/02, A61K 8/06, A61K 8/23

(54) **EYELASH ORNAMENT COSMETIC**

(30) Priority: 25.05.2016 JP 2016104591
(71) Applicant: MITSUBISHI PENCIL COMPANY, LIMITED, Tokyo 140-8537 (JP)
(72) Inventor: Hayakawa, Takayuki, Gunma, 375-8501 (JP); Sakuma, Satoshi, Gunma, 375-8501 (JP)
(74) Representative: Vossius & Partner Patentanwälte Rechtsanwälte mbB

(57) **Abstract**

Provide is an eyelash ornament cosmetic characterized by containing at least a polymer emulsion, an extender and a viscosity regulator and controlling an average particle diameter of the extender to 1 *µ*m more, wherein droplets put on the tips of eyelashes are dried and turned into fine spherical resin balls, and the tips of the eyelashes are ornamented to make it possible to provide the eyes with brilliantness and loveliness and further enhance stability of the quality.

## Description

### BACKGROUND OF THE INVENTION

### 1. Field OF THE INVENTION

### [Technical Field]

The present invention relates to a cosmetic for ornamenting the tips of eyelashes. More specifically, it relates to an eyelash ornament cosmetic which is put on the tips of the eyelashes in the form of droplets and which is dried and turned into fine spherical resin balls to make it possible to stably ornament the tips of the eyelashes.

### 2. Description of the Related Art

### [Background Art]

As a makeup method for ornamenting eyelashes, methods in which eyelashes are curled or extended by a mascara have so far been usually used as cosmetics for ornamenting eyelashes.

Known are, for example, a method in which a water-proof coating is used to thereby enhance durability of curling and a method in which eyelashes are allowed to look longer by blending fibers.

However, the methods described above intend to efficiently use or emphasize the original forms of eyelashes, and they are not to provide the eyelashes with brilliantness and loveliness.

Also, a method in which vividness is given to eyes by applying a mascara having an unusual color on eyelashes is used as well. Brilliantness can be exhibited by the method, but the situation used is limited, and the method is usually hard to use.

A cosmetic for eyelashes which is prepared by blending an alkyl acrylate copolymer emulsion, a moisturizer and a polyacrylate and which provides eyelashes with a good volume feeling by curling is known as a conventional cosmetic for eyelashes (for example, patent document 1). In the above cosmetic for eyelashes, "curling power", "smoothness" and "quick drying" are emphasized for evaluation, and the primary focus is not put on enabling specific ornament.

Further, known is a cosmetic for eyelashes which contains an acryl base film-forming polymer having a glass transition temperature of 80°C or higher and another film-forming polymer emulsion and which does not form clots (for example, patent document 2). It is described therein that the cosmetic for eyelashes contains preferably powders having an average particle diameter of 1 to 20 *µ*m and that spherical inorganic powders (spherical silica) are particularly preferred. Also in the patent document, the primary focus is not put on enabling specific ornament.

Further, a cosmetic which contains at least a polymer emulsion and an extender and which maintains a three-dimensional form after drying by controlling an average particle diameter of the extender to 1.0 µm or less is applied by the present inventors as a cosmetic which can readily provide the eyes with brilliantness and loveliness without resistance by applying a specific makeup (spherical decoration) on the eyelashes (Japanese Patent Publication No. 2016-104731).

### Related Art Documents

### Patent documents

Patent document 1: JP-A 2014-159386, (claims, examples and others)
Patent document 2: JP-A 2008-50315, (claims, paragraph [0032], examples and others)

### OUTLINE OF THE INVENTION

### [Problems to be Solved by the Invention]

However, in the cosmetic concerned, a viscosity and a color tone of the cosmetic are changed due to fluctuation in the property of the extender of 1.0 µm or less, and therefore the quality of the cosmetic becomes a little instable whenever produced. Further, a little room of improvement has been involved therein in terms of inferior workability, a high cost and difficulty of treating the residual monomer.

### SUMMARY OF THE INVENTION

In light of the existing situations of the conventional arts described above, an object of the present invention is to provide an eyelash ornament cosmetic which can easily provide eyes with brilliantness and loveliness without resistance by applying specific makeup such as spherical decoration on eyelashes and which can further improve a quality in each production, that is, a viscosity being important for forming spherical decoration and stability of a hue being important in providing eyes with loveliness.

### [Means for Solving the Problems]

The present invention relates to an eyelash ornament cosmetic which contains a polymer emulsion, an extender and a viscosity regulator and which can maintain a three-dimensional form after drying by controlling an average particle diameter of the extender to a specific value or higher and can further improve a quality in each production, that is, a viscosity and stability of a hue.

That is, the eyelash ornament cosmetic of the present invention is characterized in that it contains at least a polymer emulsion, an extender and a viscosity regulator and that an average particle diameter of the extender is controlled to 1 µm or more.

Further, it is characterized in that the extender described above comprises acrylic base resin particles.

Also, it is characterized in that a total solid content of the polymer emulsion and the extender described above is 20 to 70 % by mass based on the total amount of the cosmetic.

It is characterized in that the component of the polymer emulsion is an acryl resin emulsion.

It is characterized in that the polymer emulsion has a glass transition temperature (Tg) of 50°C or lower.

Also, it is characterized in that it has a viscosity of 1000 to 20000 mPa•s at a shear rate of 3.83 s⁻¹ at 25°C.

### [Effects of the invention]

According to the present invention, provided is an eyelash ornament cosmetic which can readily be applied on the eyelashes by the user in the form of droplets, which can be put on the eyelashes in the form of resin balls having various colors by drying the droplets to make it possible to provide the eyes with brilliantness and loveliness and which can improve stability of the quality in every manufactured products, that is, stability of a viscosity and a hue thereof.

### DETAILED DESCRIPTION OF THE INVENTION

### [Embodiment of the Invention]

The embodiments of the present invention shall be explained below in detail.

The eyelash ornament cosmetic of the present invention is characterized in that it contains at least a polymer emulsion, an extender and a viscosity regulator and that the extender has an average particle diameter of 1 *µ*m or more.

The polymer emulsion used in the present invention is added for the purpose of forming and maintaining the spherical form of the cosmetic. The "polymer emulsion" prescribed in the present invention means an emulsion which is dispersed by a surfactant and the like and has a film-forming property.

The polymer emulsion used in the present invention includes an acryl resin emulsion, a urethane resin emulsion, a vinyl acetate resin emulsion and the like.

In the above polymer emulsions, a glass transition temperature (Tg) of the polymer shall not specifically be restricted, and it is preferably 50°C or lower, more preferably 40°C or lower from the viewpoints of hardness after drying, maintaining the form and forming the spheres.

In the present invention (including examples described later), the "glass transition temperature (Tg)" means temperature at which when an amorphous solid is heated, the hard solid having no fluidity like a crystal at low temperature is reduced rapidly in rigidity and a viscosity and increased in fluidity in some narrow temperature range (temperature at which glass transition is caused).

An acryl resin emulsion is desirably used as the preferred polymer emulsion from the viewpoints of hardness and compatibility with the extender.

The acryl resin emulsion which can be used includes, for example, alkyl acrylate copolymer emulsions, acrylic ester-methacrylic ester copolymer emulsions, alkyl acrylate•styrene copolymer emulsions, vinyl acetate•alkyl acrylate copolymer emulsions, and the like.

The polymer emulsion which can specifically be used includes, in the case of the acryl resin emulsion, commercially available Yodosol GH34F (Tg: lower than 50°C; alkyl acrylate copolymer emulsion, manufactured by Akzo Nobel N.V.), Vinysol 1076DM (Tg: 50°C or higher; alkyl acrylate copolymer emulsion, manufactured by Daido Chemical Industry Co., Ltd.), Daitosol 5000AD (Tg: lower than 50°C; alkyl acrylate copolymer emulsion, manufactured by Daito Kasei Kogyo Co., Ltd.), and the like; in the case of the urethane resin emulsion, BAYCUSAN C1000 (Tg: lower than 50°C, manufactured by Bayer Material Science A.G.), BAYCUSAN C1004 (Tg: lower than 50°C, manufactured by Bayer Material Science A.G.), and the like; and in the case of the vinyl acetate resin emulsion, Vinyblan GV5651 (Tg: lower than 50°C, manufactured by Nissin Chemical Industry Co., Ltd.), and the like.

A content of the above polymer emulsions is varied in the relation to the extender used, and it is 1 to 60 % by mass (hereinafter, referred to merely as "%"), preferably 5 to 40 % in terms of a solid content based on the total amount of the cosmetic.

The extender having an average particle diameter of 1 *µ*m or more which is used in the present invention is added for the purpose of forming a spherical shape.

The extender which can be used includes, for example, at least one of the respective resin particles of acryl resins, urethane resins and the like, and talc, mica, barium sulfate, silicon dioxide (silica) and the like.

The extenders have to have an average particle diameter of 1 *µ*m or more, preferably 1 to 50 *µ*m, more preferably 1 to 20 *µ*m from the viewpoints of maintaining the form and providing the viscosity.

The acryl resin particles of the resins are desirably used as the preferred extender from the viewpoint of compatibility with the polymer emulsion.

The "average particle diameter" of the extender prescribed in the present invention means a number average particle diameter. In a method for measuring the average particle diameter, the sample (extender) is spread in a thin layer on a tape, and an enlarged image of the sample piece is photographed under a scanning electron microscope. The diameters of the sharp images of ten spherical particles present in the vicinity of the center of the enlarged image are measured, and an average value thereof is determined to set to the "average particle diameter".

The resin particles of the acrylic resins, the urethane resins and the like mean particles which do not have a film-forming property and are not dispersed.

The acryl resin particles which can be used include polymethyl methacrylate resin particles, alkyl acrylate•styrene copolymer particles and the like, and they include, to be specific, commercially available Matsumoto Microsphere M-101 (average particle diameter: 5 to 15 µm, polymethyl methacrylate resin particles, manufactured by

Matsumoto Yushi-Seiyaku Co., Ltd.), Techno Polymer MB-4C (average particle diameter: 4 µm, polymethyl methacrylate resin particles, manufactured by Sekisui Plastics Co., Ltd.), Jurymer MB-1 (average particle diameter: 7.6 µm, polymethyl methacrylate resin particles, manufactured by Toagosei Co., Ltd.), and the like.

The extenders can be used alone or in a mixture of two or more kinds thereof.

A content of the extender is adjusted in relation to the foregoing polymer emulsion used. A total content of the polymer emulsion and the extender used is 15 to 75 %, preferably 20 to 70 % and particularly preferably 20 to 60 % in terms of a solid content based on the total amount of the cosmetic from the viewpoints of the fluidity and maintaining the shape.

If a total content of the polymer emulsion and the extender used is less than 15 % in terms of a solid content, the solid matter is short, and the shape is deflated and cannot be maintained. On the other hand, if it exceeds 75 %, the fluidity is markedly gets worse, and the shape cannot be maintained. Accordingly, both are not preferred.

The viscosity regulator used in the present invention may be any ones as long as they can provide aqueous cosmetics with viscosity. For example, and xanthan gum, cellulose preparations, succinoglycan, carbomer and the like can be used alone or in a mixture of two or more kinds thereof.

The xanthan gum is a general term which is called zansan gum and santan gum in another name. It is natural high molecular polysaccharides obtained by microbial fermentation and is formed usually by repeating a bonding block comprising a constitutional unit formed by two glucoses, two mannoses and one glucuronic acid, wherein the glucuronic acid is present in the form of a potassium, sodium or calcium salt.

The xanthan gum which can be used includes, for example, commercially available "KELZAN" series, Keltrol (trade name, manufactured by Sansho Co., Ltd.) and the like. In the present invention, the "xanthan gum" may be a refined product or a modified product.

The cellulose preparation which can be used includes, for example, at least one of crystalline cellulose, fermented cellulose, oxidized cellulose, flat cellulose, and the like.

The crystalline cellulose is a cellulose crystallite aggregate substantially having a fixed polymerization degree which is obtained by subjecting cellulose to acid hydrolysis or alkali oxidative decomposition, and it has a characteristic in which cellulose crystals are turned into dispersed particles in water.

The crystalline cellulose which can be used includes, for example, cellulose comprising a colloidal grade obtained by subjecting the surfaces of fine cellulose crystalites of primary particles to coating treatment with a water-soluble polymer and the like. The specific examples thereof include Ceolus RC-591, RC-N81, RC-591NF, CL-611, Ceolus Cream (all manufactured by Asahi Kasei Corporation), and the like.

The fermented cellulose means cellulose produced by cellulose producing bacteria classified into Acetobacter genus, Pseudomonas genus, Agrobacterium genus and the like. It is produced by, for example, subjecting acetic acid bacterium to aerated and agitated culture on a suitable medium and separating and collecting cellulose fibers obtained from the bacterial cell, and it is a fibrous substance forming a very fine three-dimensional network structure in an aqueous medium.

Commercially available products may be used for the fermented cellulose. Also, fermented celluloses obtained by culturing acetic acid bacteria such as Acetobacter xylium and Acetobacter pasteurianus on a medium containing nutrients such as a nitrogen source, a carbon source, water and oxygen can be used. For example, products prepared by fermenting the flesh and the juice of coconut with cellulose producing bacteria such as the acetic acid bacteria described above and others may be used as the fermented cellulose in the present invention. The commercially available products include Sun Artist H-PX, H-PG and H-PN (all manufactured by San-Ei Gen F.F.I. Inc.), and the like.

The oxidized cellulose shall not specifically be restricted as long as it has a cellulose I type crystal structure and is modified in a part of hydroxyl groups (-OH group) of β-glucose constituting cellulose [(C₆H₁₀O₅)ₙ: natural polymer in which many β-glucose molecules are polymerized via glucoside bonds in a linear form] with at least one functional group of an aldehyde group (-CHO group) and a carboxyl group (-COOH group), and it includes, for example, oxidized celluloses obtained by oxidizing a hydroxyl group (-OH group) of the β-glucose at least at a C6 position to modify it to an aldehyde group (-CHO group) or a carboxyl group (-COOH group). A production method for the same shall not specifically be restricted as well, and commercially available products such as, for example, Leo Crista (manufactured by DKS Co., Ltd.) can be used.

The flat cellulose is flat cellulose fine particles obtained by mixing a cellulosic material such as fibrous or powdery wood flour or wood pulp originating in wood with 0.1 to 100 % by mass of fatty acids including saturated fatty acids such as stearic acid and derivatives thereof, unsaturated fatty acids such as oleic acid and derivatives thereof to obtain a mixture and subjecting the mixture to mechanical crushing treatment, and commercially available products, if available, can be used.

The succinoglycan is a kind of polysaccharides originating in microorganisms and contains a sugar unit derived from galactose and glucose and in addition thereto, a unit derived from succinic acid and pyruvic acid, and acetic acid of an optional component or salts of these acids.

The succinoglycan which can be used includes, for example, commercially available RHEOZAN SH (manufactured by Solvay Nicca Ltd.). In the present invention, the "succinoglycan" shall be a refined product or a modified product.

The carbomer (carboxyvinyl polymer) is a water-soluble polymer having a carboxyl group which comprises acrylic acid as a principal chain and contains allyl sugar and pentaerythritol as a cross-linking agent, and the commercially available products include, for example, CARBOPOL series (manufactured by BF Goodrich Corporation), HIVISWAKO series (manufactured by Wako Pure Chemical Industries, Co., Ltd.), and the like. In the present invention, the "carbomer" shall include modified products thereof, for example, alkyl-modified products such as copolymerized polymers of long-chain alkyl acrylate monomers having 10 to 30 carbon atoms with olefinically unsaturated carboxylic acid monomers.

A content of the viscosity regulators is varied depending on the kind of the viscosity regulators used, and it is preferably 0.01 to 1.00 %, particularly preferably 0.1 to 0.5 % based on the total amount of the cosmetic in terms of keeping a balance with a viscosity of the cosmetic described later and inhibiting settling of a pigment.

If a content of the viscosity regulator is less than 0.01 %, the droplets cannot be maintained on the eyelashes, and on the other hand, if it exceeds 1.00 %, the fluidity is notably deteriorated to make it impossible to form the spherical shape. Accordingly, both are not preferred.

The eyelash ornament cosmetic of the present invention contains the respective components described above and can contain, if necessary, a suitable amount of a coloring material in order to provide a three-dimensional color pattern and a color shape.

Organic pigments, inorganic pigments, pearl pigments, and other bright pigments which are usually used for cosmetics can be used as the usable coloring material.

Capable of being listed are blue color No. 1 Al lake, red color No. 202, red color No. 226, No. 228, blue color No. 404, red color No. 220, yellow color No. 401, yellow color No. 205, blue color No. 201, blue color No. 204, yellow color No. 4 Al lake, yellow color No. 203 Al lake, red color No. 104 Al lake, and the like as the organic pigments, carbon black, Prussian blue, red iron oxide, yellow iron oxide, black iron oxide, titanium oxide, and the like as the inorganic pigments, and mica, titanated mica, carmine-coated titanated mica, carmine•Prussian blue-coated titanated mica, black iron oxide-coated titanated mica, black iron oxide•carmine-coated titanated mica, black iron oxide•Prussian blue-coated titanated mica, blue-coated titanated mica, red iron oxide-coated mica, red iron oxide-coated titanated mica, red iron oxide•carmine-coated titanated mica, red iron oxide•black iron oxide-coated titanated mica, red iron oxide•Prussian blue-coated titanated mica, red iron oxide•black iron oxide•Prussian blue-coated titanated mica, and the like as the pearl pigments.

A content of the coloring material is 0 to 10 %, preferably 0 to 5 % based on the total amount of the cosmetic in order to obtain the suitable three-dimensional color pattern, color shape and brilliantness.

Also, the eyelash ornament cosmetic of the present invention can contain water (refined water, distilled water, ion-exchanged water, purified water and the like) as the balance. Further, it can contain a water-soluble organic solvent for the purpose of controlling the drying property, and other optional components, for example, a pH controlling agent, a neutralizing agent, a UV absorber, a UV scattering agent, a moisturizing agent, a perfume, an antioxidant, an antiseptic agent, a sequestering agent, a defoaming agent, various extracts, and other various kinds of components.

The organic solvent which can be used includes, for example, alcohols such as ethanol, phenoxyethanol and isopropanol, glycols such as 1,3-butylene glycol and pentylene glycol and glycerins such as glycerin and ethylhexyl glycerin.

A content of the organic solvents is 0 to 20 %, preferably 0.1 to 20 % based on the total amount of the cosmetic in order to obtain the suitable drying property.

The eyelash ornament cosmetic of the present invention has a viscosity of preferably 1000 to 50000 mPa•s, more preferably 1000 to 20000 mPa•s and particularly preferably 1500 to 5000 mPa•s at a shear rate of 3.83 s⁻¹ at 25°C from the viewpoint of forming the three-dimensional shape.

If the viscosity is less than 1000 mPa•s, the droplets cannot be held on the eyelashes, and on the other hand, if it exceeds 50000 mPa•s, the spherical droplets cannot be formed in certain cases.

The viscosity ranges described above can be controlled by suitably combining the polymer emulsion, the extender, the viscosity regulator, water and the like each described above and effectively combining them in the ranges of the contents described above.

The eyelash ornament cosmetic of the present invention can be prepared by an ordinary method, and it is filled in an applicator for eyelashes and applied on the eyelashes.

Capable of being used are applicators such as, for example, brushes prepared by winding a wire around fibers usually used to fix them, applicators of a comb shape and a coil shape, resin-molded brushes molded a resin to a brush form, brushes, flocks, and rod-like applicators.

The eyelash ornament cosmetic of the present invention thus constituted contains at least the polymer emulsion, the extender and the viscosity regulator, and an average particle diameter of the extender is controlled to 1 *µ*m or more, so that the user can readily apply droplets on eyelashes. Further, the droplets can be dried to put the resin spheres of various colors on the eyelashes to result in obtaining an eyelash ornament cosmetic which can provide the eyes with brilliantness and loveliness and which can be further enhanced in stability of the quality by containing the viscosity regulator.

### EAMPLES

Next, the present invention shall be explained in further details with reference to examples, but the present invention shall not be restricted by the following examples and others.

### Examples 1 to 9 and Comparative Examples 1 to 3

The components were mixed and dispersed according to recipes (total amount: 100 %) shown in the following Table 1 by means of a planetary stirring device, a planetary mixer, a homomixer or a disper to produce (prepare) the respective eyelash ornament cosmetics.

In respective Examples 1 to 9 and Comparative Examples 1 to 3, the respective eyelash ornament cosmetics (products) were produced each three times, and the respective eyelash ornament cosmetics produced first time were set to the standard products.

The respective eyelash ornament cosmetics obtained in Examples 1 to 9 and Comparative Examples 1 to 3 were used to evaluate the hues and measure the respective viscosities at a shear rate of 3.83 s⁻¹ at 25°C by the following measuring methods. Further, the adhesiveness onto eyelashes, the three-dimensional shape-forming property and the quality stability were evaluated by the following evaluation methods. The results thereof are shown in the following Table 1.

### Evaluating method of hue:

The respective eyelash ornament cosmetics were filled in a screw cap bottle SV-10 (thickness: 1.3 mm, outer diameter 24 mm x height 45 mm, volume 10 ml, manufactured by Nichiden-Rika Glass Co., Ltd.), and the bottles were covered with caps and left still standing one day at a temperature of 25°C. Then, the bottles were inspected from an outside to observe a change in the hues, that is, the presence of a change in the hues of the respective eyelash ornament cosmetics produced second time and third time was observed based on the respective standard products produced first time.
S : Standard
N : No change in hue
PG : Colored pale green
G : Colored green

### Measuring method of viscosity:

The respective eyelash ornament cosmetics obtained above were used to measure a viscosity at the prescribed shear rate at a temperature of 25°C by means of a cone plate type viscometer (among TV-30 type viscometers, an EHD type viscometer, a standard cone plate, manufactured by Tokimec Inc.).

### Evaluation methods of adhesiveness onto eyelashes and three-dimensional shape-forming property:

The respective eyelash ornament cosmetics were charged into an exclusive vessel, applied on eyelashes and dried to evaluate an adhesiveness onto the eyelashes and a three-dimensional shape-forming property according to the following respective evaluation criteria. They were evaluated by one exclusive panel.

### Evaluation criterion of adhesiveness onto the eyelashes:

⊚: Spheres applied on eyelashes were not removed all day
○: Spheres applied on eyelashes were not removed all day but readily removed by touch with a finger
Δ: Spheres applied on eyelashes were removed in daily life ×: Spheres were not adhered on eyelashes

### Evaluation criterion of three-dimensional shape-forming property:

⊚: Fine spherical ornaments were always formed after drying
○: Three-dimensional ornaments having any form were always formed after drying
Δ: Three-dimensional ornaments having any form were partially formed after drying
×: Three-dimensional ornaments were not formed after drying

### Evaluation methods of quality stability:

The three products of the respective eyelash ornament cosmetics were compared with each other in the respective examples or comparative examples to evaluate the quality stability according to the following evaluation criteria.

### Evaluation criterion of quality stability:

⊚: Products having a leveled and stabilized quality are obtained in the products of the respective examples or comparative examples
○: Fluctuation in quality is observed in an allowable range in the products of the respective examples or comparative examples
Δ: Large fluctuation in quality is observed in the products of the respective examples or comparative examples
X: Quality is not stabilized at all (quality is different every time) in the products of the respective examples or comparative examples.

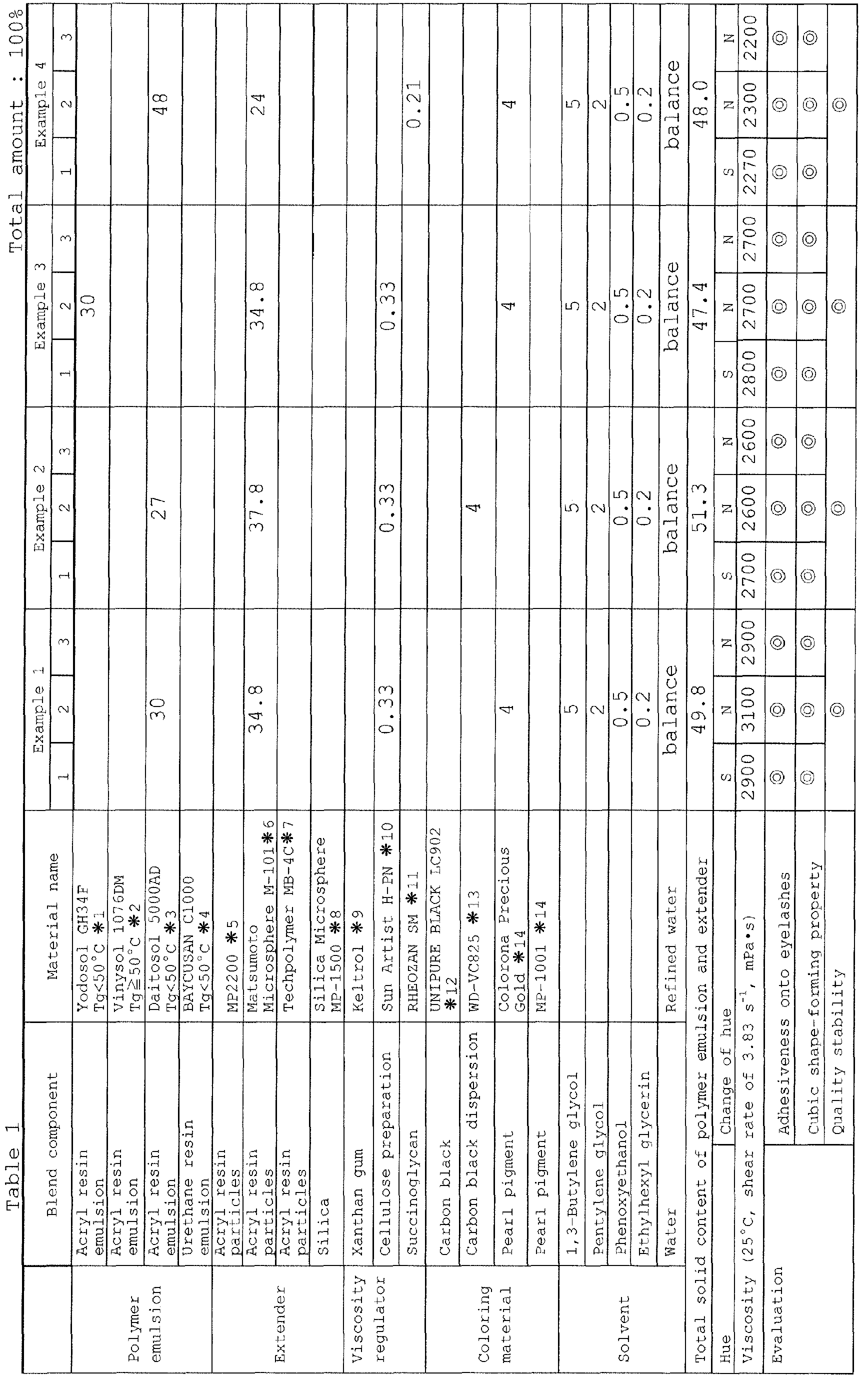

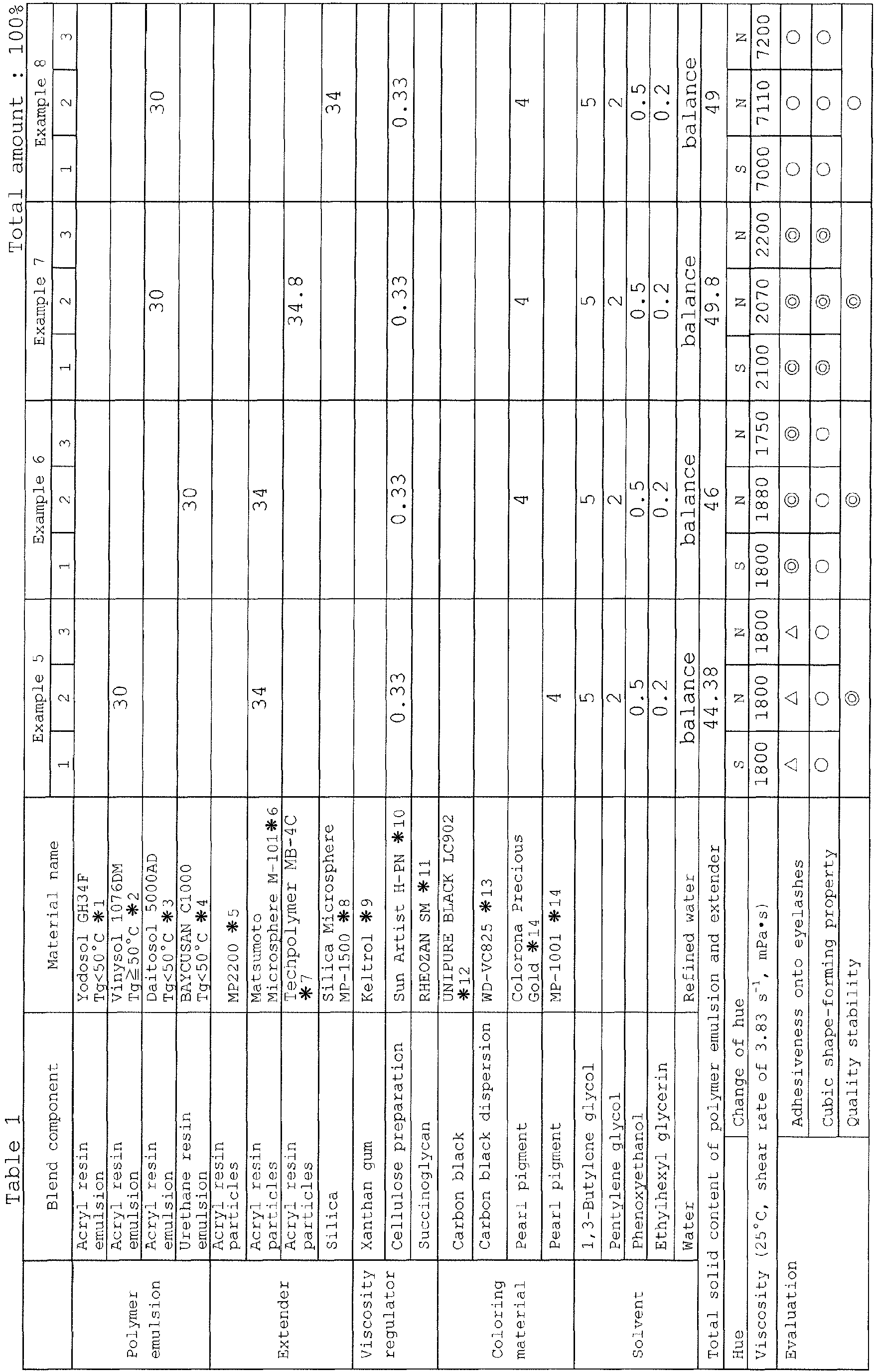

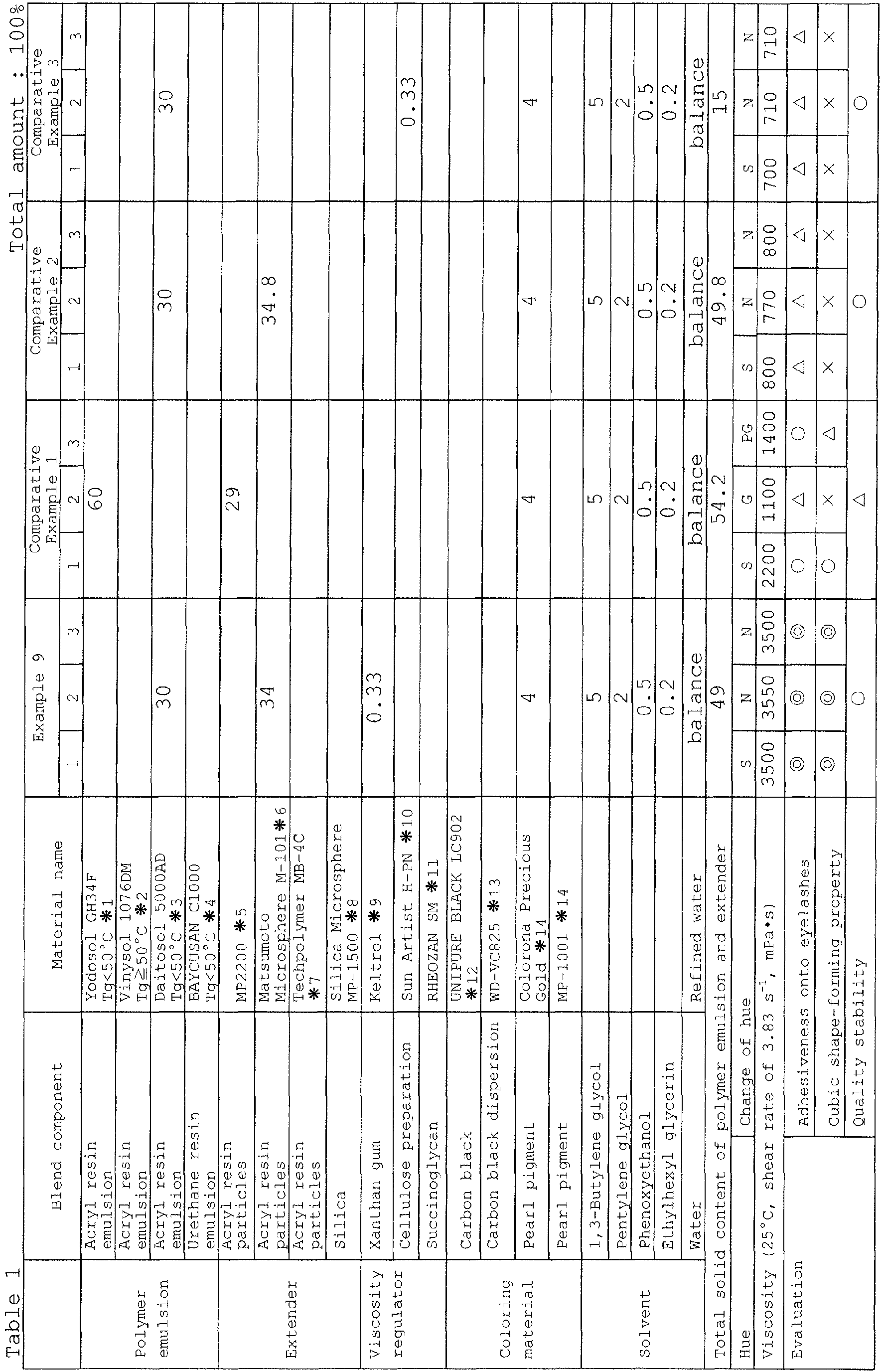

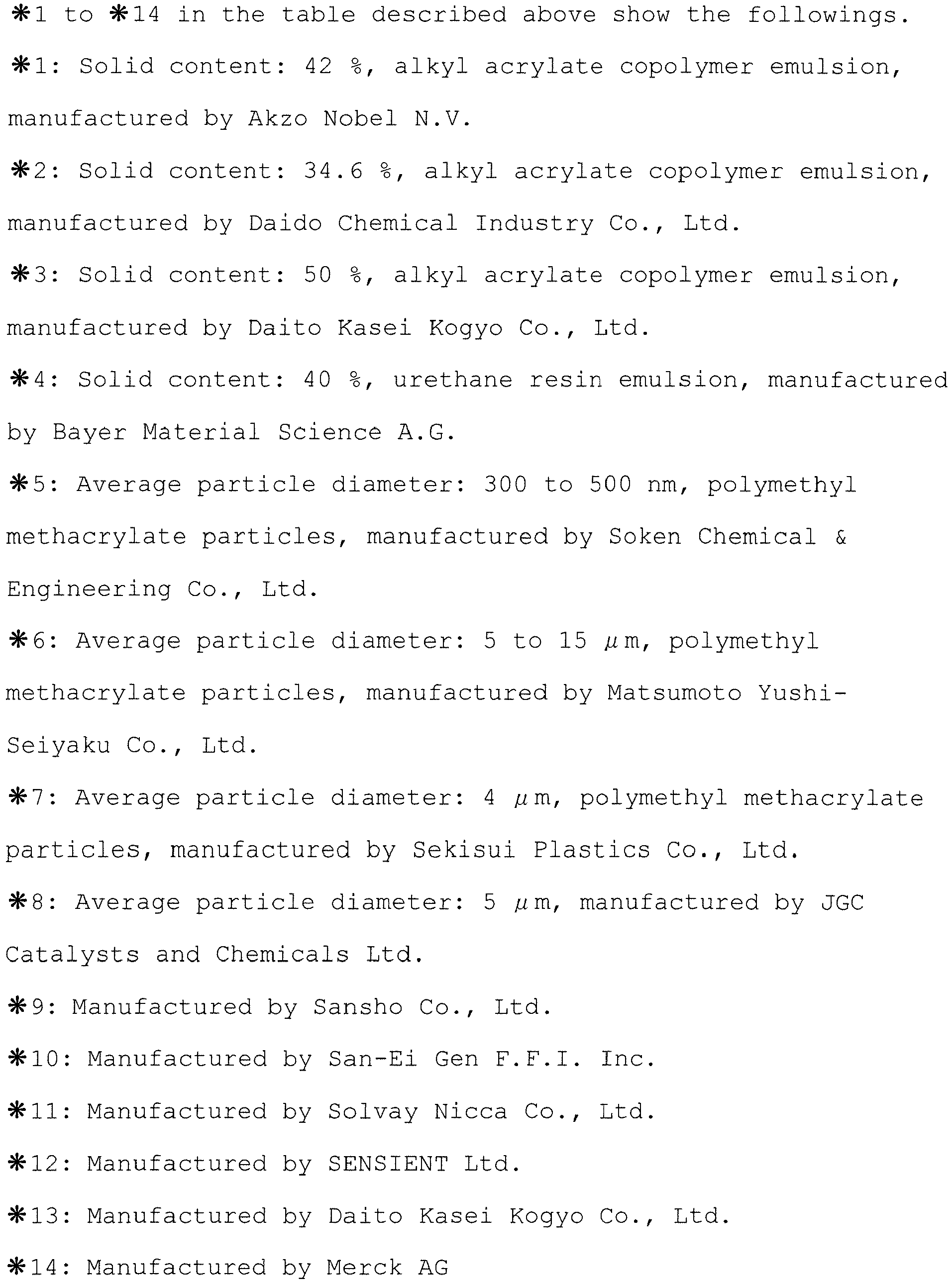

As apparent from the results summarized in Table 1 shown above, it has been found clear that the eyelash ornament cosmetics falling in the scope of the present invention which were prepared in Examples 1 to 9 are excellent in adhesiveness onto eyelashes, a three-dimensional shape-forming property and quality stability in producing three times as compared with the eyelash ornament cosmetics falling outside the scope of the present invention which were prepared in Comparative Examples 1 to 3.

To observe individually the comparative examples, in Comparative Example 1, the viscosity regulator was not contained, and the extender having an average particle diameter of less than 1 µm was used; in Comparative Example 2, the viscosity regulator was not contained as compared with Example 1; and in Comparative Example 3, the extender was not contained as compared with Example 7. It has been found that in the above cases, the effects of the present invention cannot be exerted.

To further study Comparative Examples 1 to 3 shown above, it has been estimated, though the reasons therefor are not clear, that in Comparative Example 1 in which the extender having a small average particle diameter of less than 1 µm was used, the eyelash ornament cosmetics having a stable quality, that is, those which are fixed in a viscosity and a hue cannot be produced because of the reasons that the extender particles having a smaller particle diameter than the average particle diameter are diverse in a shape and that they are not uniform in the condition of the surfaces. In Comparative Example 2, the eyelash ornament cosmetics contain the extender particles which have an average particle diameter of 1 µm or more and which are relatively uniform in a shape and the condition of the surfaces, but they do not contain the viscosity regulator, and therefore it is estimated that the compositions have a low viscosity and require too much time for exerting a three-dimensional shape-forming property to result in finally making it impossible to form a three-dimensional shape on the eyelashes. In the case of Comparative Example 3, the extender is not contained, and therefore it is estimated that a three-dimensional shape cannot be formed on the eyelashes.

### [Industrial Applicability]

An eyelash ornament cosmetic which can readily provide the eyelashes with ornaments is obtained.

## Claims

1. An eyelash ornament cosmetic **characterized by** containing at least a polymer emulsion, an extender and a viscosity regulator and controlling an average particle diameter of the extender to 1 *µ*m or more.

2. The eyelash ornament cosmetic as described in claim 1, wherein the extender is acryl resin particles.

3. The eyelash ornament cosmetic as described in claim 1 or 2, wherein a total solid content of the polymer emulsion and the extender is 20 to 70 % by mass based on the total amount of the cosmetic.

4. The eyelash ornament cosmetic as described in any one of claims 1 to 3, wherein the polymer emulsion is an acryl base polymer emulsion.

5. The eyelash ornament cosmetic as described in any one of claims 1 to 4, wherein the polymer in the polymer emulsion has a glass transition temperature (Tg) of 50°C or lower.

6. The eyelash ornament cosmetic as described in any one of claims 1 to 5, wherein the cosmetic has a viscosity of 1000 to 20000 mPa•s at a shear rate of 3.83 s⁻¹ at 25°C.
